# EUROPEAN PATENT APPLICATION

(11) **EP 1 229 071 A1**
(43) Date of publication of application: **07.08.2002**
(21) Application number: 00955024.5
(22) Date of filing: 25.08.2000
(51) Int. Cl.: C08J 11/26, C08G 18/83, C07C 29/88

(54) **METHOD OF TREATING POLYOL RECOVERED THROUGH DECOMPOSITION AND POLYOL RECOVERED THROUGH DECOMPOSITION**

(30) Priority: 16.09.1999 JP 26134699; 16.09.1999 JP 26134799; 28.10.1999 JP 30669699
(71) Applicant: MITSUI TAKEDA CHEMICALS, INC., Tokyo (JP); Kabushiki Kaisha Kobe Seiko Sho (Kobe Steel, Ltd.), Kobe-shi, Hyogo 651-8585 (JP); HONDA MOTOR CO., Ltd., Tokyo 107-8556 (JP)
(72) Inventor: KODAMA, Katsuhisa, E-232, 11-35, Otanicho, Hyogo 662-0054 (JP); MURAYAMA, Kouichi, c/o R&D Center of Mitsui Takeda, Yodogawa-ku, Osaka-shi Osaka 532-8686 (JP); KUMAKI, Takashi, c/o R&D Center of Mitsui Takeda, Yodogawa-ku, Osaka-shi Osaka 532-8686 (JP)
(74) Representative: Weber, Thomas, Dr.Dipl.-Chem.
(86) International application number: JP0005748
(87) International publication number: WO0119907

(57) **Abstract**

A method for processing decomposed reusable polyol, which method is used for processing the decomposed and recovered polyol obtained by decomposing polyurethane resin, and the decomposed reusable polyol obtained by the processing method. In the processing method, any of the following processes is selectively used: the process that after an organic dicarboxylic acid or anhydride thereof is added to the decomposed and recovered polyol containing the amines, the precipitate is removed; the process that an isocyanate compound is added to the decomposed and recovered polyol containing the amines; the process that an oxide compound is added to the decomposed and recovered polyol containing the amines; and the process that a urea is added to the decomposed and recovered polyol containing the amines.

## Description

### Technical Field

This invention relates to a method for processing decomposed reusable polyol and decomposed reusable polyol and, more particularly, to a method for processing decomposed reusable polyol obtained by decomposing polyurethane resin and to the as-decomposed reusable polyol obtained by the processing method of the present invention.

### Background Art

In recent years, from the standpoints of global environmental conservation and resource conservation, recycling of plastic material is being desired increasingly. This applies to polyurethane resins, such as flexible polyurethane foams used as cushioning material for seats of vehicles and for furniture items and rigid polyurethane foams used as heat insulating material for houses and for refrigerators and the like. Various types of recycling methods, including a material recycling method and a chemical recycling method, are being studied and some are being put in practical use.

For example, an alkali decomposing process, a glycolysis process (a glycol decomposing process), an aminolysis process (an amine decomposing process) and a hydrolysis process are known as the chemical recycling method for polyurethane resin. The glycolysis process using low molecular weight glycol for decomposition of polyurethane resin has partly come into practical use.

However, with the alkali decomposing process, the aminolysis process and the hydrolysis process, polyamine, an intermediate material of polyisocyanate which is a starting material, and an amine compound used as a decomposer are allowed to dissolve in polyol decomposed and recovered. As a result of this, when the decomposed and recovered polyol containing these dissolved amines is reused as raw material of polyurethane, normal reaction of polyol and polyisocyanate can be hindered to cause production of inferior goods.

Accordingly, the alkali decomposing process, the aminolysis process and the hydrolysis process are intended to include a distilling operation and equivalent to remove the amines from the decomposed and recovered polyol obtained. The distilling operation and equivalent enables the amines to be removed in some degree but does not enable the amines to be removed completely. In usual, the distilling operation allows some weight percent of amines to exist in the decomposed and recovered polyol. Accordingly, there were proposed the processes of neutralizing and filtrating the decomposed and recovered polyol, for example, by washing the decomposed and recovered polyol with water containing mineral acid such as hydrochloric acid or by blowing hydrochloric acid gas into the decomposed and recovered polyol (Cf. Japanese Laid-open Unexamined Patent Publication Nos. Sho 55-86814 and Sho 57-80438). However, the former suffers from the disadvantage of having difficulty in separating polyol from a water layer because polyol has in general a hydrophilic nature, and the latter suffers from the disadvantage of having difficulty in crystallizing the salt of amine produced and thus difficulty in separation by filtration. For these reasons, those methods have not yet been put into practice successfully.

On the other hand, it is known that the flexible polyurethane foams used for seats of vehicles can easily be nearly completely decomposed to polyol which is a starting material and polyamine which is an intermediate material of polyisocyanate which is a starting material via the hydrolysis using high temperature and high pressure water of 250-320 °C (Cf. International Publication No. WO98/34904, for example). When the decomposed materials thus obtained are subjected to a vacuum distillation process, the polyamine can be recovered in some degree. But, a small amount of polyamine still remains in the decomposed and recovered polyol, so that the decomposed and recovered polyol is high in basicity so that it cannot be practically reused as it stands as the raw material of the urethane resin. It is known that this method may also be combined with the process of neutralizing the polyamine remaining in the decomposed and recovered polyol to remove the polyamine as the salt by use of mineral acid such as hydrochloric acid. But, the salt of polyamine is difficult to crystallize and thus is difficult to separate by filtration, as mentioned above.

When the salt of polyamine remains in the decomposed and recovered polyol, the acid value of polyol increases, so that, when the decomposed and recovered polyol is reused as the raw material for urethane resin, there arises a problem of a delay in the reaction to form urethane.

Accordingly, it is, for the present, an important subject for study on how to process the amines in the decomposed and recovered polyol which is obtained by the chemical recycling methods, such as the alkali decomposing process, the aminolysis process and the hydrolysis process, to obtain the decomposed reusable polyol that can be reused as the raw material of polyurethane resin.

It is an object of the present invention to provide a method for processing decomposed reusable polyol, according to which the amines contained in the decomposed and recovered polyol are inactivated with or without being removed with a simple process, to obtain the reusable polyol that can be adequately reused as the raw material of polyurethane resin, and to provide decomposed reusable polyol obtained by the process of the same method.

### Disclosure of the Invention

The present invention provides a method for processing decomposed polyol, wherein decomposed and recovered polyol containing amines obtained by decomposition of polyurethane resin is further subjected to processing to remove the amines and recover the decomposed and recovered polyol therefrom as reusable products. This process provides the result that when the decomposed and recovered polyol is reused as the raw material of polyurethane resin, the reaction to form urethane is not hindered by the amines and, accordingly, the polyol can be reused as the polyol of high quality in a variety of fields.

According to the present invention, it is preferable that after an organic dicarboxylic acid or anhydride thereof is added to the decomposed and recovered polyol containing the amines, the precipitate is removed, thereby subjecting the amines to processing. This process using the organic dicarboxylic acids or anhydrides thereof enables the precipitate to be produced with ease, as compared with the process using hydrochloric acid and, hence, the amines can be removed with ease and efficiency to thereby produce the polyol of high quality. Besides, the organic dicarboxylic acid or anhydride thereof does not hinder the reaction to form urethane so much, as compared with hydrochloric acid and, therefore, even if it remains, the polyol can well be reused as the raw material of polyurethane resin. Further, when the removed precipitate is disposed, little harmful substances may be produced, differently from chlorine containing compound derived from chlorine or hydrochloric acid, and, hence, there is little possibility of contributing the environmental pollution.

In this process, it is preferable that the organic dicarboxylic acid or anhydride thereof is oxalic acid and also it is preferable that the organic dicarboxylic acid or anhydride thereof is added to the decomposed and recovered polyol containing the amines in such a proportion that the organic dicarboxylic acid or anhydride thereof contains 0.3-1.05 equivalents of carboxyl group per equivalent of amino group of the amines.

According to the present invention, an isocyanate compound is added to the decomposed and recovered polyol containing the amines, thereby subjecting the amines to processing. This process enables the amines to be inactivated as they are without removing them by simply adding the isocyanate compound to the decomposed and recovered polyol containing the amines. Thus, this process can be put into practice industrially and with an easy and simple way. Besides, this process provides the result that when the decomposed and recovered polyol obtained by this process is reused as the raw material of polyurethane resin, the reaction to form urethane is not hindered by the amines and also the acid value is prevented from increasing, differently from the neutralizing process using the acid. Hence, the polyol obtained by this process can be reused as the polyol of high quality in a variety of fields.

In this process, it is preferable that the isocyanate compound is added in such a proportion that the isocyanate compound contains 0.8-1.5 equivalents of isocyanate group per equivalent of amino group of the amines in the decomposed and recovered polyol containing the amines.

According to the present invention, it is preferable that an oxide compound is added to the decomposed and recovered polyol containing the amines, thereby subjecting the amines to processing. This process enables the amines to be inactivated as they are without removing them by simply adding the oxide compound to the polyol decomposed and recovered. Thus, this process can be put into practice industrially and with an easy and simple way. Besides, this process provides the result that when the polyol obtained by this process is reused as the raw material of urethane resin, the reaction to form urethane is not hindered by the amines and also the acid value is prevented from increasing, differently from the neutralizing process using the acid. Hence, the polyol obtained by this process can be reused as the polyol of high quality in a variety of fields.

In this process, it is preferable that the oxide compound is added to the decomposed and recovered polyol containing the amines in the presence of no catalyst. It is also preferable that the oxide compound is added to the decomposed and recovered polyol containing the amines and heated at 80-140°C. In addition, it is preferable that the oxide compound is added in such a proportion that the oxide compound contains 1.5-3.0 equivalents of oxide group per equivalent of amino group of the amines in the decomposed and recovered polyol containing the amines.

According to the present invention, it is preferable that a urea is added to the decomposed and recovered polyol containing the amines, thereby subjecting the amines to processing. This process enables the amines to be inactivated as they are without removing them by simply adding the urea to the polyol decomposed and recovered. Thus, this process can advantageously be put into practice simply and industrially. Besides, this process provides the result that when the decomposed and recovered polyol obtained by this process is reused as the raw material of urethane resin, the reaction to form urethane is not hindered by the amines. Hence, the polyol obtained by this process can be reused as the polyol of high quality in a variety of fields.

In this process, it is preferable that the urea is added in the proportion of 0.4-1.5 equivalents per equivalent of amino group of the amines in the decomposed and recovered polyol containing the amines.

According to the present invention, it is preferable that the decomposed and recovered polyol containing the amines is polyether polyol. Also, it is preferable that the content of the amines in the decomposed and recovered polyol containing the amines is 5 weight % or less.

The method for processing decomposed polyol of the present invention is applied to a recovering process of a decomposing and recovering process of polyurethane resin comprising a hydrolyzing process for hydrolyzing the polyurethane resin and the recovering process for recovering the decomposed product produced by the hydrolysis.

The present invention covers a decomposed reusable polyol obtained by performing the process that after an organic dicarboxylic acid or anhydride thereof is added to the decomposed and recovered polyol containing the amines which is obtained by decomposition of polyurethane resin, the precipitate is removed; a decomposed reusable polyol obtained by performing the process that an isocyanate compound is added to the decomposed and recovered polyol containing the amines which is obtained by decomposition of polyurethane resin; a decomposed reusable polyol obtained by performing the process that an oxide compound is added to the decomposed and recovered polyol containing the amines which is obtained by decomposition of polyurethane resin; and a decomposed reusable polyol obtained by performing the process that urea is added to decomposed and recovered polyol containing amines which is obtained by decomposition of polyurethane resin.

When these decomposed reusable polyols are reused as the raw material of polyurethane resin, the reaction to form urethane is not hindered by the amines. Hence, the decomposed reusable polyols can be reused as the polyol of high quality in a variety of fields.

### Brief Description of the Drawings

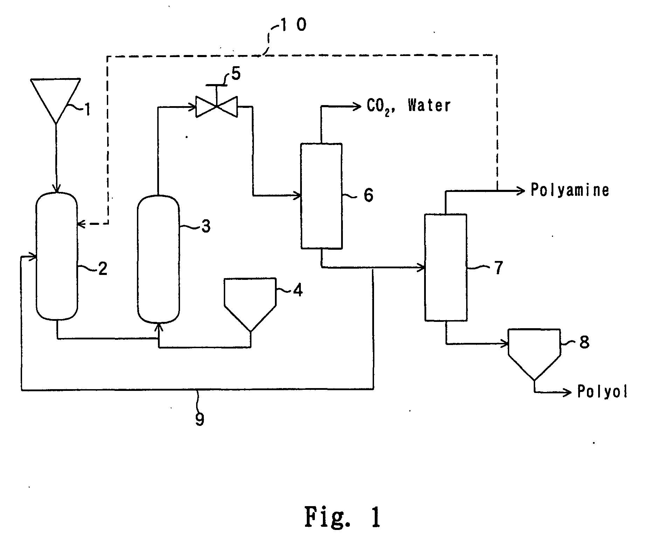

FIG. 1 is a schematic diagram showing an embodied form of an apparatus for use in industrially practicing a decomposing and recovering method for polyurethane resin to which the method for processing decomposed polyol of the present invention is applied.

### Best Mode for Carrying out the Invention

The decomposed and recovered polyol to which the processing method of the present invention is applicable are the polyol obtained by decomposition of polyurethane resin and containing amines therein.

The polyurethane resins to be decomposed are synthetic polymer compounds obtained by the reaction of polyol and polyisocyanate, including, such as flexible, semi-rigid or rigid polyurethane foams and cast or thermoplastic polyurethane elastomer. The polyurethane resins can be decomposed by a known method without being limited to any particular method. For example, the chemical recycling methods which may be used include an alkali decomposing process, a glycolysis process (a glycol decomposing process), an aminolysis process (an amine decomposing process), a hydrolysis process and a combination thereof.

Polyols include compounds having at least two or more hydroxyl groups that are starting material of polyurethane resins. For example, low molecular weight polyols and high molecular weight polyols can be cited.

The low molecular weight polyols include, for example, low molecular weight diols, such as ethylene glycol, propylene glycol, 1,4-butylene glycol, 1,6-hexane diol, neopentyl glycol, diethylene glycol, triethylene glycol, dipropylene glycol, bisphenol A, hydrogenated bisphenol A, and xylene glycol, low molecular weight triols, such as glycerine and 1,1,1-tris (hydroxy methyl) propane, and low molecular polyols having at least four hydroxyl groups, such as D-sorbitol, xylitol, D-mannitol, and D-mannit.

The high molecular weight polyols include, for example, polyether polyol (polyoxyalkylene polyol), polyester polyol, polycarbonate polyol, acrylic polyol, epoxy polyol, natural oil polyol, silicon polyol, fluorine polyol and polyolefine polyol.

Of these polyols, the high molecular weight polyols, particularly the high molecular polyols of number average molecular weight of the order of 800-20,000, are preferable as the polyols to which the processing method of the present invention is applicable. Among others, polyether polyol is of preferable.

Examples of polyether polyol are polyoxyethylene glycol and/or polyoxypropylene glycol including random copolymer and/or block copolymer) which are obtained, for example, by addition reaction of alkylene oxides, such as ethylene oxide and/or propylene oxide, with an initiator having at least two active hydrogen groups, and polytetramethylene ether glycol obtained, for example, by ring opening polymerization of tetrahydrofuran.

The amines contained in the polyol include, for example, polyamine produced as an intermediate material of polyisocyanate together with polyol by decomposition of polyurethane resin, and an amine compound used as a decomposer when decomposing the polyurethane resin by the aminolysis process.

The polyamines as the intermediate material of polyisocyanate include compounds having at least two or more amino groups, which include, for example, aromatic diamines, such as diaminodiphenyl methan (MDA) that is an intermediate material of diphenylmethane diisocyanate (MDI) and tolylene diamine (TDA) that is an intermediate material of tolylene diisocyanate (TDI), aralkyl diamines, such as xylylene diamine (XDA) that is an intermediate material of xylylene diisocyanate (XDI) and tetramethyl xylylene diamine (TMXDA) that is an intermediate material of tetramethyl xylylene diisocyanate (TMXDI), alicyclic diamines, such as 3-aminomethyl-3,5,5-trimethylcyclohexyl amine (IPDA) that is an intermediate material of 3-isocyanatomethyl-3,5,5-trimethylcyclohexyl isocyanate (IPDI), 4,4'-methylene-bis (cyclohexyl amine) (H₁₂MDA) that is an intermediate material of 4,4'-methylene-bis (cyclohexyl isocyanate) (H₁₂MDI), and bis (aminomethyl) cyclohexane (H₆XDA) that is an intermediate material of bis (isocyanatomethyl) cyclohexane (H₆XDI), aliphatic diamines, such as hexamethylene diamine (HDA) that is an intermediate material of hexamethylene diisocyanate (HDI), and polymethylene polyphenyl polyamine that is an intermediate material of polymethylene ponyphenyl polyisocyanate (crude MDI, polymeric MDI).

The amine compounds which may be used as the decomposer in the aminolysis process include, for example, mono-amino compounds; but equivalent to the polyamines listed above can preferably be used.

Specifically, the decomposed and recovered polyols containing the amines include, for example, a mixture of the polyol, which is a starting material of the urethane resin and is obtained by decomposition of the polyurethane resin, and polyamine, which is an intermediate material of the polyisocyanate that is also the starting material, and further a mixture of those and the amine compound used as the decomposer.

In the processing method of the present invention, the amines are preferably contained in the polyol of interest in the proportion of not more than 5 weight %, preferably not more than 4 weight %, or further preferably 0.1 to 3 weight %. If the amines are contained in the polyol in the proportion in excess of those specific values, then an amine derivative produced by the process of the processing method of the present invention will increase so much that there is the possibility that the polyol cannot be reused as raw material, depending on the intended use.

In the processing method of the present invention, the decomposed and recovered polyol containing the amines obtained by the decomposition of the polyurethane resin is subjected to processing, so that the amines are processed so that the decomposed and recovered polyol can be reused. Specifically, the amines are preferably processed by use of any process selected from those consisting of the process that after an organic dicarboxylic acid or anhydride thereof is added to the decomposed and recovered polyol containing the amines, the precipitate is removed; the process that an isocyanate compound is added to the decomposed and recovered polyol containing the amines; the process that an oxide compound is added to the decomposed and recovered polyol containing the amines; and the process that a urea is added to the decomposed and recovered polyol containing the amines. The conditions of these various types of processes may be suitably determined in accordance with the conditions for the polyol to be decomposed and recovered.

The processing method of the present invention is preferably applied particularly to a recovering process of the decomposing and recovering process of polyurethane resin comprising a fluidizing process for fluidizing polyurethane resin, a hydrolyzing process for hydrolyzing the fluidized polyurethane resin, and the recovering process for recovering a decomposed product produced by the hydrolysis.

In the following, a further specific description will be given on the processing method of the present invention, taking the application to the decomposing and recovering process for polyurethane resin as an example. With reference to FIG. 1, in which there is shown an embodied form of an apparatus for use in industrially practicing this decomposing and recovering process, the processing method of the present invention will be described. It is to be noted that in FIG. 1 of a schematic diagram, incidental facilities, such as a pump and a heater, are omitted.

The polyurethane resins, for which the decomposing and recovering method of the present invention is used, include, for example, flexible, semi-rigid or rigid polyurethane foams and cast or thermoplastic polyurethane elastomer, as mentioned above. To be more specific, off-cuts and chips of those urethane resins, produced when shaped in the form of various types of domestic or industrial products, and waste articles thereof are used. Even -when some other materials, such as fibers, leathers, synthetic leathers or metals, are contained in those products, they have no objection to the method of the present invention, but they are preferably cut or ground to a specified size properly so that they can be easily processed.

First, in the fluidizing process, the polyurethane resin thrown from a hopper 1 used as throwing means is fluidized in a fluidization tank 2. The fluidizing processes include, for example, a aminolysis process in which an amino compound is allowed to act on the polyurethane resin, a slurrying process in which the polyurethane resin is dispersed in a dispersion medium with physical stirring, and a solubilization process in which the polyurethane resin is dissolved by solvent. The aminolysis process is preferably used.

In the aminolysis process, the polyurethane resin is added into a liquefied amine compound and heated to about 120°C to about 220°C, preferably about 150°C to about 200°C, to fluidize the polyurethane resin. With a lower heating temperature than this, it may take longer to fluidize the polyurethane resin. On the other hand, with a higher temperature than that, decomposition or polymerization of the amine compound may occur so that the polyurethane resin cannot be fluidized. Preferably, the amine compound used as the decomposer in the aminolysis is the polyamine that is produced after the polyurethane resin is hydrolyzed. When this polyamine is used under reflux, the decomposition and recovery after hydrolyzation can be facilitated and also reduction of costs can be achieved. Further, a polyol compound may be blended in the amine compound. The blend of the polyol compound enables viscosity in a system to be lowered to provide uniform fluidization. When the amine compound and the polyol compound are used in combination, they are preferably blended in the proportion of 0.5-5 parts by weight of polyol compound per part by weight of amine compound. With a higher proportion of formulation of the polyol compound than that, there is the possibility that the polyurethane resin may not be well fluidized. The polyol compound used is preferably the polyol that is produced after the polyurethane resin is hydrolyzed, for the same reason as mentioned above.

To be more specific, a reflux line 9 is provided, as shown in FIG. 1, between an intermediate part of a line extending between a dehydration tank 6 and a separation tank 7 and the fluidization tank 2 as mentioned later so that the mixture of the polyamine and polyol that are produced after the polyurethane resin is hydrolyzed can be refluxed into the fluidization tank 2 to provide the aminolysis. Alternatively, a reflux line 10 may be provided between a polyamine recovering line at the downstream side of the separation tank 7 and the fluidization tank 2 so that only the polyamine can be refluxed. Further, those reflux liens 9 and 10 may be used in combination.

The polyurethane resin thus fluidized is then hydrolyzed in the hydrolysis process. When some fibers or metals are mixed in the polyurethane resin fluidized in the fluidizing process, a filtration means and the like is preferably used, if required, to remove the fibers or metals before the transition to the hydrolysis process, through not shown in FIG. 1.

The hydrolysis process is performed in a hydrolyzing tank 3, using, for example, supercritical water or high temperature and high pressure water fed from a feed-water tank 4, at 200-400°C, preferably 250-320°C, under the pressure in excess of a pressure under which water can be kept in its liquid state in that temperature range. With a lower temperature than that, the decomposition rate may reduce, while on the other hand, with a higher temperature than that, there is the possibility that decomposition or side reaction of polyol or polyamine produced may occur. The weight of water used is preferably 0.3-10.0 parts by weight per part by weight of the fluidized polyurethane resin (hereinafter it is referred to as "the addition-of-water ratio"). Further preferably, the addition-of-water ratio is in the range of 0.3 to 5.0. With a lower addition-of-water ratio than that, there is possibility that the decomposition may be incomplete, while on the other hand, with a higher addition-of-water ratio than that, an energy loss may increase to lead to waste of energy. In the hydrolysis process, a small amount of alkali metal hydroxide or ammonia may be used as a catalyst.

The hydrolysis enables the fluidized polyurethane resin to be decomposed to polyol which is the starting material and polyamine which is an intermediate mateiral of polyisocyanate which is the starting material.

Sequentially, the decomposed products obtained are separated and recovered in the recovering process. Before that, they are preferably subjected to a dehydration process to remove the water used in the hydrolysis process and carbon dioxide produced in the hydrolysis process. The dehydration and degasification in the dehydration process may be performed in the dehydration tank 6 by use of any of the known methods such as single distillation, flash distillation, reduced-pressure distillation, absorption and drying. The flash distillation is preferably used. The flash distillation enables the water and the carbon dioxide, which are under pressure in the hydrolysis process, to be evaporated under reduced pressure by simply using a pressure-regulating valve 5 and equivalent to throw open them under atmospheric pressure.

Then, in the recovering process, polyol and amines are separated and recovered from the mixture of the polyol and the amines from which the water and carbon dioxide are removed (the amines are mainly polyamine which is the intermediate material of polyisocyanate which is the starting material, including an amine compound used as decomposer in the aminolysis process). The processing method of the present invention is applied in this process.

While the mixture of the polyol and the amines may be directly processed in the recovering process by using the processing method of the present, it is preferable that the mixture is roughly separated into crude polyol and crude amines in a separating process, first, and then the crude polyol thus obtained is processed in the next process using the processing method of the present invention.

The rough separation of the polyol and the amines in the separating process can be performed in the separation tank 7 by any known method such as distillation, extraction, centrifugation, absorption and drying. The distillation is preferably used for recovering high-molecular-weight polyol. The distillation enables the crude amines and the crude polyol to be efficiently separated into a light fraction in boiling and a heavy fraction in boiling, respectively. In the separating process, the rough separation should preferably be performed so that the content of the amines in the polyol can be 5 weight % or less, as aforementioned.

With a content of the amines more than 5 weight %, the derivatives of amines in the polyol after processed will increase, so that the polyol may not be reused, depending on the intended uses, as aforementioned.

In the processing process, the crude polyol, i.e., the polyol containing the amines, is processed in a processing tank 8 by using the processing method of the present invention. This process is performed by selectively using any of the following processes: the process that after for example an organic dicarboxylic acid or anhydride thereof is added to the decomposed and recovered polyol containing the amines, the precipitate is removed; the process that for example an isocyanate compound is added to the decomposed and recovered polyol containing the amines; the process that for example an oxide compound is added to the decomposed and recovered polyol containing the amines; and the process that for example a urea is added to the decomposed and recovered polyol containing the amines.

In the process that after an organic dicarboxylic acid or anhydride thereof is added to the decomposed and recovered polyol containing the amines, the precipitate is removed, the amines are allowed to precipitate as the salt of the organic dicarboxylic acid by adding the dicarboxylic acid or anhydride thereof to the polyol containing the amines and then the precipitate is removed.

The organic dicarboxylic acids or anhydrides thereof which may be used in this process include, for example, oxalic acid, succinic acid, maleic acid and maleic anhydride. Preferable are oxalic acid and maleic anhydride. Further preferable is oxalic acid. The organic dicarboxylic acids or anhydrides thereof are preferably added in such a proportion that the organic dicarboxylic acid or anhydride thereof contains 0.3-1.05, preferably 0.5-1.03, or further preferably 0.7-1.01, equivalents of carboxyl group per equivalent of amino group of the amines. With the addition of less than those equivalents, the salt is difficult to precipitate or a required amount of amines may not precipitate, such that the amines cannot be removed completely. On the other hand, with the addition of more than those equivalents, the organic dicarboxylic acid or anhydride thereof may remain in the polyol after processed, such that products of high quality may not be obtained. For allowing the salt to precipitate, the decomposed and recovered polyol is preferably heated to 50-140°C under a nitrogen atmosphere.

The precipitate is removed by a known process such as filtration and centrifugation. Filtration is preferably used. The filtration enables the precipitate to be successively removed with a simple device.

The polyol obtained by this process in which the amines are nearly completely removed can be reused as the polyol of high quality in a variety of fields. In this process method using the organic dicarboxylic acid or anhydride thereof, the salt is crystallized and precipitated with ease, as compared with the process using hydrochloric acid and, hence, the amines can be removed with ease and efficiency to thereby produce the polyol of high quality. Besides, the organic dicarboxylic acid or anhydride thereof does not hinder the reaction to form urethane so much, as compared with hydrochloric acid and, therefore, there is a little possibility of hindering the reaction to form urethane when the polyol thus produced is reused. Further, when the removed salt of the amines is disposed, there is little possibility that harmful substances may be produced, differently from chlorine, and, hence, there is little possibility of contributing the environmental pollution.

In the process that the isocyanate compound is added to the decomposed and recovered polyol containing the amines, when the isocyanate compound is added to the polyol containing the amines, the isocyanate group of the isocyanate compound reacts with the amino group of the amines to produce polyuria compound, whereby the amines are inactivated.

Although the isocyanate group of the isocyanate compound can also be allowed to react with hydroxyl group of the polyol, since reactivity of the amino group to the isocyanate group is higher than reactivity of the hydroxyl group to the isocyanate group, when the isocyanate compound is added to the polyol containing the amines, the isocyanate group of the isocyanate compound reacts with the amino group of the amines selectively.

The isocyanate compounds used are compounds having at least one isocyanate group, which include, for example, a mono-isocyanate compounds such as phenyl isocyanate, aromatic diisocyanates, such as diphenylmethan diisocyanate (MDI) and tolylene diisocyanate (TDI), aralkyl diisocyanates, such as xylylene diisocyanate (XDI) and tetramethyl xylylene diisocyanate (TMXDI), alicyclic diisocyanates, such as 3-isocyanatomethyl-3,5,5-trimethylcyclohexyl isocyanate (IPDI), 4,4'-methylene-bis (cyclohexyl isocyanate) (H₁₂MDI), and bis (isocyanatomethyl) cyclohexane (H₆XDI), and aliphatic diisocyanates, such as hexamethylene diisocyanate (HDI), all of which are used as raw material of urethane resin. The isocyanate compounds may be used singularly or in combination of two or more and may be selectively used in accordance with the conditions of decomposition, separation and recovery or the intended use for reuse.

The isocyanate compound is preferably added in such a proportion that the isocyanate compound contains 0.8-1.5, or preferably 0.9-1.3, equivalents of isocyanate group per equivalent of amino group of the amines in the polyol. With the addition of less than those equivalents, a remaining amount of the amines having the amino group that is not allowed to react with the isocyanate compound increases. As a result of this, when the polyol is reused as the raw material of polyurethane resin, normal reaction of the polyol and polyisocyanate may be hindered to cause production of inferior goods. On the other hand, with the addition of more than those equivalents, the isocyanate group of the isocyanate compound may react with the polyol exceedingly to cause deterioration in quality of polyol.

It is preferable that the amount of isocyanate compound to be added is allowed to vary properly in accordance with the proportion of primary hydroxyl group and secondary hydroxyl group contained in the polyol as well. The primary hydroxyl group is higher in reactivity with the isocyanate group than the secondary hydroxyl group. Hence, for example, when the content of the primary hydroxyl group in the polyol is more than that of the secondary hydroxyl group, the proportion of the isocyanate group to the amino group should preferably be increased. On the other hand, when the content of the secondary hydroxyl group in the polyol is more than that of the primary hydroxyl group, the proportion of the isocyanate group to the amino group should preferably be decreased (for example, the equivalence ratio is set in the neighborhood of 1.0). Decision on which number of hydroxyl group is contained more in the polyol can be made, for example, from the types of polyurethane resin to be decomposed.

Specifically, in this process, the isocyanate compound can be added to the crude polyol in the above-noted proportion while stirring the crude polyol in the processing tank 8. In order to reduce the viscosity of the polyol and terminate the reaction of isocyanate compound and amines in a short time, this process may preferably be performed, for example, at 50-100°C, though it may be performed at room temperature.

This process enables the amines to be inactivated as they are without removing them by simply adding the isocyanate compound to the polyol decomposed and recovered. Thus, this process can advantageously be put into practice simply and industrially. Besides, this process provides the result that when the polyol obtained by this process is reused as the raw material of urethane resin, the reaction to form urethane is not hindered by the amines and also the acid value is prevented from increasing, differently from the neutralizing process using the acid. Hence, the polyol obtained by this process can be reused as the polyol of high quality in a variety of fields.

The polyol obtained by this process comes to have the property of polyuria compound, which is produced by the reaction of the amines and the isocyanate compound, being dispersed in the polyol (polyuria compound dispersed polyol). This property of the polyuria compound being dispersed has no particular influence to the quality of polyol. Also, the property can contribute to the uses for e.g. polymer dispersed polyol, depending on the proportion of the polyuria compound contained in the polyol obtained and the dispersed state.

In the process that an oxide compound is added to the decomposed and recovered polyol containing the amines, when the oxide compound is added to the decomposed and recovered polyol containing the amines, it undergoes ring opening reaction with the amino group of the amines to form the hydroxyl group (alkanolamine), whereby the activity of the amines are reduced.

Although oxide group of the oxide compound can also be allowed to react with the hydroxyl group of the polyol, since reactivity of the amino group to the oxide group is higher than reactivity of the hydroxyl group to the oxide group, when the oxide compound is added to the polyol containing the amines, the oxide group of the oxide compound react with the amino group of the amines selectively.

The oxide compounds used are compounds having at least one oxide group (epoxy group, or an atomic group -O- that bonds to carbon of two atomic elements bonded to each other via a chain). Any known oxide compounds may be used without any particular limitation. For example, alkylene oxides, such as ethylene oxide, propylene oxide and butylene oxide, styrene oxide, or phenyl glycidyl ether are used. These oxide compounds may be used singularly or in combination of two or more and may selectively be used in accordance with the conditions of decomposition, separation and recovery or the intended use for reuse. Of these oxide compounds, the alkylene oxides are preferably used. Among others, the ethylene oxide and the propylene oxide are preferably used.

The oxide compound is preferably added in such a proportion that the oxide compound contains 1.5-3.0, or preferably 2.0-2.5, equivalents of oxide group per equivalent of amino group of the amines in the polyol. With the addition of less than those equivalents, the activity of the amines cannot be reduced sufficiently, so that when the polyol is reused as the raw material of polyurethane resin, the normal reaction of the polyol and the polyisocyanate may be hindered to cause production of inferior goods. On the other hand, with the addition of more than those equivalents, the oxide group of the oxide compound may be exceedingly added to the hydroxyl group of oxide-adducts of the amines or the hydroxyl group of polyol, to cause deterioration in quality of polyol.

Specifically, in this process, the oxide compound can be added to the crude polyol in the above-noted proportion while stirring the crude polyol in the processing tank 8. This process is preferably performed in the presence of no catalyst. In the presence of no catalyst, the oxide group of the oxide compound, the hydroxyl group of the polyol and the hydroxyl group of the oxide-adducts of the amines produced by this process are not allowed to substantially react with each other, so that the selectivity of the oxide group of the oxide compound to the amino group of the amines can be improved further to allow the reaction of the oxide compound and the amines to progress further quantitatively.

Also, it is preferable in this process that after the oxide compound is added to the crude polyol, the polyol is heated at 80-140°C, or preferably at 100-130°C. The heating enables the viscosity of the polyol to be reduced and also enables the reaction of the oxide group of the oxide compound and the amino group of the amines to be accelerated, to shorten the time for the reaction to be terminated. In addition, in order to accelerate the process, the pressure is preferably applied in the order of 0.05-0.5MPa, for example, though the process may of course be performed under normal pressure.

This process enables the amines to be inactivated as they are without removing them by simply adding the oxide compound to the polyol decomposed and recovered. Thus, this process can advantageously be put into practice simply and industrially. Besides, this process provides the result that when the polyol obtained by this process is reused as the raw material of polyurethane resin, the reaction to form urethane is not hindered by the amines and also the acid value is prevented from increasing, differently from the neutralizing process using the acid. Hence, the polyol obtained by this process can be reused as the polyol of high quality in a variety of fields.

The polyol obtained in this process is the mixture of the polyol and the oxide-adducts of the amines produced by the reaction of the amines and the oxide compound. Also, the OH value of the polyol obtained is increased to an extent corresponding to the addition of the oxide compound, as compared with the polyol before processed (for example, supposing that 1 weight % of tolylene diamine is contained in the polyol, when 1 mole of propylene oxide is added to the tolylene diamine, the OH value is increased to about 3.1 on calculation. Supposing that 5 weight % of tolylene diamine is contained in the polyol, when 4 moles of propylene oxide is added to the tolylene diamine, the OH value is increased to about 32 on calculation). However, these have no particular influence to the quality of polyol. The polyol obtain may be used as it is as the polyol comprising the mixture of polyoxyalkylene polyol of a polyol base initiator, and polyoxyalkylene polyol of an amine base initiator, which have been prescribed, for example, for the use for heat insulating material of electric refrigerators, depending on the composition and the proportion of the oxide-adducts of the amines in the polyol obtained.

In the process that a urea is added to the decomposed and recovered polyol containing the amines, when the urea is added to the polyol containing the amines, exchange reaction occurs between the urea and the amino group of the amines, so that urea derivatives are produced by deammonification and thereby the amines are inactivated.

The urea is preferably added in the proportion of 0.4-1.5, or preferably 0.5-1.2, equivalents of urea per equivalent of amino group of the amines in the polyol. With the addition of less than those equivalents, a remaining amount of the amines having the amino group that is not allowed to react with the urea increases. As a result of this, when the polyol is reused as the raw material of urethane resin, normal reaction of the polyol and polyisocyanate may be hindered to cause production of inferior goods. On the other hand, with the addition of more than those equivalents, an excessive amount of non-reacted urea may be crystallized to cause deterioration in quality of polyol.

Specifically, in this process, the urea can be added to the crude polyol in the above-noted proportion while stirring the crude polyol in the processing tank 8. This process is preferably performed under heating. Preferably, this process is performed, for example, at 120-200°C, or further preferably at 130-180°C. With a processing temperature lower than that, there is the possibility that the reaction of the urea and the amino group of the amines may not progress. On the other hand, with a processing temperature higher than that, there is the possibility that the urea itself may be decomposed. This process entails the production of ammonia gas, so that it is preferable that the ammonia gas is removed from the processing tank 8 (reactive), for example, by blowing nitrogen gas into the processing tank 8 or reducing the pressure in the processing tank 8.

This process enables the amines to be inactivated as they are without removing them by simply adding the urea the polyol decomposed and recovered. Thus, this process can advantageously be put into practice simply and industrially. Besides, this process provides the result that when the polyol obtained by this process is reused as the raw material of urethane resin, the reaction to form urethane is not hindered by the amines. Hence, the polyol obtained by this process can be reused as the polyol of high quality in a variety of fields.

The polyol obtained by this process comes to have the property of urea derivative, which is produced by the reaction of the amines and the urea, being dispersed in the polyol (urea derivative dispersed polyol). This may produce turbidity or a crystal precipitating state, depending on the quantity of amines in the polyol before processed or the quantity of urea used. For this case, the processing tank 8 may be provided with a filter or like means for removing the turbidity and the precipitated crystals to remove them. The turbidity and the precipitated crystals have no particular influence to the quality of polyol. Accordingly, the polyol having this property of urea derivative being dispersed in the polyol may be used for e.g. polymer dispersed polyol, without removing the turbidity and the precipitated crystals, depending on the intended purposes and uses.

In the foregoing, there are illustrated various processes of the present invention, including the process that after an organic dicarboxylic acid or anhydride thereof is added to the decomposed and recovered polyol containing the amines, the precipitate is removed; the process that an isocyanate compound is added to the decomposed and recovered polyol containing the amines; the process that an oxide compound is added to the decomposed and recovered polyol containing the amines; and the process that a urea is added to the decomposed and recovered polyol containing the amines, taking as an example the application to the recovering process of the decomposing and recovering process for polyurethane resin comprising the fluidizing process, the hydrolyzing process and the recovering process. These various processes can be applied, for example, to any of the alkali decomposing process, the glycolysis process (glycol decomposing process), the aminolysis process (amine decomposing process) and other known processes as mentioned above, without limiting to that decomposing and recovering process for polyurethane resin. Also, no limitation is imposed on the kinds of decomposed objects, i.e., the kinds of polyurethane resins, the kinds of polyols and the kinds of amines.

### Examples

In the following, the present invention will be described further concretely with reference to Examples and Comparative Examples.

### A: Process using an organic dicarboxylic acid or anhydride thereof:

### Fluidizing Process:

50g of tolylene diamine and 50g of polyoxypropylene triol (number average molecular weight of 3,000) were charged in a 500ml four-necked flask with a thermometer, an agitator, and a nitrogen gas in-lead tube and heated up to 170°C from outside. Then, 30g of off-cuts of flexible polyurethane foam of density of 25kg/m³ foamed by use of tolylene diisocyanate (TAKENATE80 available from Takeda Chemical Industries, Ltd.) and polyoxypropylene triol (number average molecular weight of 3,000) were added into the liquid and stirred for 1 hour at the same temperature to fluidize (solubilize) the foam completely. The liquid was cooled down to 25°C and the viscosity of the liquid was measured. The measurement showed that the liquid has the viscosity of about 10,000mPa · s.

### Hydrolysis Process:

Sequentially, 50g of foam dissolved solution thus obtained and 50g of purified water were charged in an autoclave of an internal volume of 200ml having a thermometer and a pressure gauge. After the substitution by nitrogen gas, they were heated up to 270°C from outside and were allowed to stand for 20 minutes. Thereafter, the autoclave was cooled down to room temperature and then the hydrolyzed product obtained was analyzed by use of GPC. It was found from the analysis result that the hydrolyzed product was decomposed to tolylene diamine (TDA) and polyoxypropylene triol of number average molecular weight of 3,000.

### Recovering Process:

In order to remove the TDA from polyoxypropylene triol (number average molecular weight of 3,000) in the hydrolyzed product, simple distillation was performed under reduced pressure. Specifically, after distilling water completely at 110°C, the hydrolyzed product was charged in a distilling apparatus flowing cooling water of 90°C therethrough, first. Then, after the pressure in the distilling apparatus was reduced to 2,000 Pa, the temperature was increased. After a low boiling fraction of the hydrolyzed product was distilled off, transparent liquid was distilled when the temperature of the distilling apparatus reached 155°C. When the temperature of the distilling apparatus reduced down to a temperature at which the liquid was no longer distilled, the simple distillation was completed. The concentration of the residue of TDA was measured by potentiometric titration using 0.1 mole/l of perchloric acid acetic acid solution. It was found therefrom that the TDA was distilled off to 2.0 weight %.

### Processing Process (Examples 1-4, Comparative Examples 1-5):

After the residue of the simple distillation obtained in the above-mentioned recovering process was put in the four-necked flask and increased in temperature up to 70°C, various kinds of acids of Examples 1-4 and Comparative Examples 1-5 shown in TABLE 1 were each added in the proportion of 0.5 and/or 1.0 times equivalents of those acids per equivalent of amino group of the TDA. Thereafter, while the nitrogen gas was blown in, the temperature was gradually increased to perform the dehydration at 120°C. One hour later, the reaction was stopped and the temperature was decreased down to 70°C, the precipitate was filtered with a 300 mesh woven metal screen.

**TABLE 1**

| | Acid | Equivalent | Precipitate | Filtration |
|---|---|---|---|---|
| Example 1 | Oxalic acid (30wt% of water) | 0.5 | Presented | Possible |
| Example 2 | Oxalic acid | 1.0 | Presented | Possible |
| Example 3 | Maleic anhydride | 0.5 | Presented | Possible |
| Example 4 | Maleic anhydride | 1.0 | Presented | Possible |
| Comparative Example 1 | Hydrochloric acid (6 mol/l) | 0.5 | Presented | Impossible |
| Comparative Example 2 | Hydrochloric acid (6 mol/l) | 1.0 | Presented | Impossible |
| Comparative Example 3 | Acetic acid | 1.0 | Not presented | Impossible |
| Comparative Example 4 | Formic acid | 1.0 | Not presented | Impossible |
| Comparative Example 5 | Acetic anhydride | 1.0 | Not presented | Impossible |

As shown in TABLE 1, the acetic acid, formic acid and acetic anhydride produced no precipitate, so that the TDA could not be removed from the system.

The oxalic acid aqueous solution, acetic anhydride and 6 mol/l of hydrochloric acid became clouded and caused the salts to precipitate. Although the 6 mol/l of hydrochloric acid caused the salts to precipitate, the size of particles of the salts were so fine that they could not be filtered with the 300 mesh woven metal screen. The centrifugation was also tried with 10,000min⁻¹ for ten minutes, almost no salts were precipitated. In contrast to this, the addition of oxalic acid aqueous solution and maleic anhydride caused sediments to precipitate as the salts and also the size of particles of the salts were large so that they were able to be filtered with ease.

### B: Process using an isocyanate compound:

### Fluidizing Process:

1,000g of tolylene diamine was charged in a 2,000ml four-necked flask with a thermometer, an agitator, and a nitrogen gas in-lead tube and heated up to 170°C from outside. Then, 600g of flexible polyurethane foam of density of 25kg/m³ foamed by use of tolylene diisocyanate (TAKENATE80 available from Takeda Chemical Industries, Ltd.) and polyoxypropylene triol (number average molecular weight of 3,000) was added into the liquid and stirred for 1 hour at the same temperature to dissolve the polyurethane foam completely.

### Hydrolysis Process:

Sequentially, 1,000g of polyurethane foam dissolved solution thus obtained and 1,000g of purified water were charged in an autoclave of an internal volume of 4,000ml having a thermometer and a pressure gauge. After the substitution by nitrogen gas, they were heated up to 270°C from outside and were allowed to stand for 20 minutes. Thereafter, the autoclave was cooled down to room temperature and then the water was stripped by nitrogen gas at 120°C. The content was diluted with methanol and was analyzed by use of GPC. It was found from the analysis result that no high-molecular-weight compound was found in the region of polyol, except the peak corresponding to the polyoxypropylene triol of number average molecular weight of 3,000, and only the peak corresponding to tolylene diamine was found in the region of amine. Also, the NMR analysis result showed that no urethane bond and no urea bond existed in the decomposed product.

### Recovering Process:

Then, the decomposed liquid thus obtained underwent a thin film distillation with 2,000Pa at 280°C, to remove tolylene diamine and recover polyoxypropylene triol as residual liquid. The content of tolylene diamine in the residual liquid after the removal of the tolylene diamine was 2.2 weight %, the amine value of the residual liquid was 20mgKOH/g, and the viscosity was 580mPa · s/25°C.

### Processing Process 1 (Example 5):

100g of polyoxypropylene triol recovered in the above-mentioned recovering process was charged in a 500ml four-necked flask with a thermometer, an agitator, and a nitrogen gas in-lead tube and heated up to 65°C. Then, 4.4g of tetramethyl xylylene diisocyanate (TMXDI) (NCO/NH₂ equivalent ratio of 1.0) was dropped in 16 minutes. Thereafter, it was aged at 65°C for 1 hour and then the reaction was terminated. The viscosity of the polyoxypropylene triol after reacted was 1,300mPa · s/25°C and the amine value was 3.0mgKOH/g, so that the amine value could be reduced to a level at which no problem was presented with the foaming when this polyoxypropylene triol was reused as the raw material of polyurethane foam.

### Processing Process 2 (Example 6):

100g of polyoxypropylene triol recovered in the above-mentioned recovering process was charged in the 500ml four-necked flask with the thermometer, the agitator, and the nitrogen gas in-lead tube and heated up to 65°C. Then, 4.0g of bis (isocyanatomethyl) cyclohexane (H₆XDI) (NCO/NH₂ equivalent ratio of 1.2) was dropped in 7 minutes. Thereafter, it was aged at 65°C for 1 hour and then the reaction was terminated. The viscosity of the polypropylene triol after reacted was 3,000mPa · s/25°C and the amine value was 4.4mgKOH/g, so that the amine value could be reduced to a level at which no problem was presented with the foaming when this polyoxypropylene triol was reused as the raw material of polyurethane foam.

### C: Process using an oxide compound:

### Fluidizing Process:

500g of tolylene diamine was charged in the 2,000ml four-necked flask with the thermometer, the agitator, and the nitrogen gas in-lead tube and heated up to 170°C from outside. Then, 300g of flexible polyurethane foam of density of 25kg/m³ foamed by use of tolylene diisocyanate (TAKENATE80 available from Takeda Chemical Industries, Ltd.) and polyoxypropylene triol (number average molecular weight of 3,000) was added into the liquid and stirred for 1 hour at the same temperature to dissolve the polyurethane foam completely.

### Hydrolysis Process:

Sequentially, 500g of polyurethane foam dissolved solution thus obtained and 500g of purified water were charged in an autoclave of an internal volume of 2,000ml having a thermometer and a pressure gauge. After the substitution by nitrogen gas, they were heated up to 270°C from outside and were allowed to stand for 20 minutes at that temperature. Thereafter, the autoclave was cooled down to room temperature and then the water was stripped by nitrogen gas at 120°C. The content was diluted with methanol and was analyzed by use of GPC. It was found from the analysis result that no high-molecular-weight compound was found in the region of polyol, except the peak corresponding to the polyoxypropylene triol of number average molecular weight of 3,000, and only the peak corresponding to tolylene diamine was found in the region of amine. Also, the NMR analysis result showed that no urethane bond and no urea bond existed in the decomposed product.

### Recovering Process:

Then, the decomposed liquid thus obtained underwent a thin film distillation with 2,000Pa at 280°C, to remove tolylene diamine and recover polyoxypropylene triol as residual liquid. The OH value of the residual liquid after the removal of the tolylene diamine was 55mgKOH/g, the content of tolylene diamine was 2.2 weight %, the amine value was 20mgKOH/g, and the viscosity was 580mPa · s/25°C.

### Processing Process (Example 7):

100g of polyoxypropylene triol (residual liquid) recovered in the above-mentioned recovering process was charged in an 500ml autoclave with a thermometer, an agitator, and a cooling tube and heated up to 120°C. The internal pressure was regulated to 0.2MPa and 5.1g of propylene oxide (oxide group/amino group equivalent ratio of 2.5) was continuously added for 1 hour by use of a pump. Thereafter, it was aged at 120°C for 30 minutes and then unreacted propylene oxide was distilled off in a stream of nitrogen.

This reaction causes the polyoxypropylene triol after the strip of propylene oxide to increase the weight by 2.2g. The OH value of polyoxypropylene triol thus obtained was 65mgKOH/g and thus increased by 10mgKOH/g, as compared with the one before processed. The viscosity was 600mPa · s/25°C. It was found from these property values that the polyoxypropylene triol after processes was of reusable as the raw material of polyurethane foam.

### D: Process using urea:

### Fluidizing Process:

500g of tolylene diamine was charged in the 2,000ml four-necked flask with the thermometer, the agitator, and the nitrogen gas in-lead tube and heated up to 200°C from outside. Then, 300g of flexible polyurethane foam of density of 25kg/m³ foamed by use of tolylene diisocyanate (TAKENATE80 available from Takeda Chemical Industries, Ltd.) and polyoxypropylene triol (number average molecular weight of 3,000) was added into the liquid and stirred for 1 hour at the same temperature to dissolve the polyurethane foam completely.

### Hydrolysis Process:

Sequentially, 500g of polyurethane foam dissolved solution thus obtained and 500g of purified water were charged in the autoclave of the internal volume of 2,000ml having the thermometer and the pressure gauge. After the substitution by nitrogen gas, they were heated up to 300°C from outside and were allowed to stand for 20 minutes. Thereafter, the autoclave was cooled down to room temperature and then the water was stripped by nitrogen gas at 120°C. The content was diluted with methanol and was analyzed by use of GPC. It was found from the analysis result that no high-molecular-weight compound was found in the region of polyol, except the peak corresponding to the polyoxypropylene triol of number average molecular weight of 3,000, and only the peak corresponding to tolylene diamine was found in the region of amine. Also, the NMR analysis result showed that no urethane bond and no urea bond existed in the decomposed product.

### Recovering Process:

Then, the decomposed liquid thus obtained underwent a thin film distillation with 2,000Pa at 280°C, to remove tolylene diamine and recover polyoxypropylene triol as residual liquid. The content of tolylene diamine in the residual liquid after the removal of the tolylene diamine was 2.2 weight %, the amine value of the residual liquid was 20mgKOH/g, and the viscosity was 580mPa · s/25°C.

### Processing Process (Example 8):

100g of polyoxypropylene triol recovered in the above-mentioned recovering process was charged in the 500ml four-necked flask with the thermometer, the agitator, and the nitrogen gas in-lead tube and heated up to 80°C. Then, 2.5g of urea (H₂NCONH₂/NH₂ equivalent ratio of 1.2) was added thereto. Thereafter, it was allowed to react under reduced pressure at 130°C for 3 hours and then underwent the nitrogen stripping for 2 hours to completely remove ammonia produced. After the termination of reaction, it was cooled down to room temperature. The viscosity of the polyoxypropylene triol after reacted was 800mPa · s/25°C and the amine value was 3.1mgKOH/g, so that the amine value could be reduced to a level at which no problem was presented with the foaming when this polypropylene triol was reused as the raw material of polyurethane foam.

While the illustrative embodiments and examples of the present invention are provided in the above description, such is for illustrative purpose only and it is not to be construed restrictively. Modification and variation of the present invention that will be obvious to those skilled in the art is to be covered by the following claims.

### Capabilities of Exploitation in Industry

As mentioned above, the method for processing decomposed reusable polyol of the present invention is suitably used for processing the decomposed and recovered polyol obtained by decomposing polyurethane resin. To be more specific, the method of the present invention can be suitably applied to the recovering process of the decomposing and recovering process of polyurethane resin comprising the hydrolyzing process for hydrolyzing the polyurethane resin and the recovering process for recovering the decomposed product produced by the hydrolysis.

## Claims

1. A method for processing decomposed polyol, wherein decomposed and recovered polyol containing amines obtained by decomposition of polyurethane resin is further subjected to processing to remove the amines and recover the decomposed and recovered polyol therefrom as reusable products.

2. The method for processing decomposed polyol according to Claim 1, wherein after an organic dicarboxylic acid or anhydride thereof is added to the decomposed and recovered polyol containing the amines, the precipitate is removed, thereby subjecting the amines to processing.

3. The method for processing decomposed polyol according to Claim 2, wherein the organic dicarboxylic acid or anhydride thereof is oxalic acid.

4. The method for processing decomposed polyol according to Claim 2, wherein the organic dicarboxylic acid or anhydride thereof is added to the decomposed and recovered polyol containing the amines in such a proportion that the organic dicarboxylic acid or anhydride thereof contains 0.3-1.05 equivalents of carboxyl group per equivalent of amino group of the amines.

5. The method for processing decomposed polyol according to Claim 1, wherein an isocyanate compound is added to the decomposed and recovered polyol containing the amines, thereby subjecting the amines to processing.

6. The method for processing decomposed polyol according to Claim 5, wherein the isocyanate compound is added in such a proportion that the isocyanate compound contains 0.8-1.5 equivalents of isocyanate group per equivalent of amino group of the amines in the decomposed and recovered polyol containing the amines.

7. The method for processing decomposed polyol according to Claim 1, wherein an oxide compound is added to the decomposed and recovered polyol containing the amines, thereby subjecting the amines to processing.

8. The method for processing decomposed polyol according to Claim 7, wherein the oxide compound is added to the decomposed and recovered polyol containing the amines in the presence of no catalyst.

9. The method for processing decomposed polyol according to Claim 7, wherein the oxide compound is added to the decomposed and recovered polyol containing the amines and heated at 80-140°C.

10. The method for processing decomposed polyol according to Claim 7, wherein the oxide compound is added in such a proportion that the oxide compound contains 1.5-3.0 equivalents of oxide group per equivalent of amino group of the amines in the decomposed and recovered polyol containing the amines.

11. The method for processing decomposed polyol according to Claim 1, wherein a urea is added to the decomposed and recovered polyol containing the amines, thereby subjecting the amines to processing.

12. The method for processing decomposed polyol according to Claim 11, wherein the urea is added in the proportion of 0.4-1.5 equivalents per equivalent of amino group of the amines in the decomposed and recovered polyol containing the amines.

13. The method for processing decomposed polyol according to any of Claims 1, 2, 5, 7 and 11, wherein the decomposed and recovered polyol containing the amines is polyoxyalkylene polyol.

14. The method for processing decomposed polyol according to any of Claims 1, 2, 5, 7 and 11, wherein the content of the amines in the decomposed and recovered polyol containing the amines is 5 weight % or less.

15. The method for processing decomposed polyol according to any of Claims 1, 2, 5, 7 and 11, which is applied to a recovering process of a decomposing and recovering process of polyurethane resin comprising a hydrolyzing process for hydrolyzing the polyurethane resin and the recovering process for recovering the decomposed product produced by the hydrolysis.

16. A decomposed reusable polyol obtained by performing the process that after an organic dicarboxylic acid or anhydride thereof is added to decomposed and recovered polyol containing amines which is obtained by decomposition of polyurethane resin, the precipitate is removed.

17. A decomposed reusable polyol obtained by performing the process that an isocyanate compound is added to decomposed and recovered polyol containing amines which is obtained by decomposition of polyurethane resin.

18. A decomposed reusable polyol obtained by performing the process that an oxide compound is added to decomposed and recovered polyol containing amines which is obtained by decomposition of polyurethane resin.

19. A decomposed reusable polyol obtained by performing the process that urea is added to decomposed and recovered polyol containing amines which is obtained by decomposition of polyurethane resin.
